(19) Europäisches Patentamt
European Patent Office
Office européen des brevets

(11) Publication number: **0 468 882 B1**

# (12) EUROPEAN PATENT SPECIFICATION

(45) Date of publication of patent specification: **22.02.95**

(51) Int. Cl.⁶: **C07C 69/88**, C09K 19/20

(21) Application number: **91402053.2**

(22) Date of filing: **24.07.91**

## (54) Liquid crystal compound.

(30) Priority: **26.07.90 JP 198500/90**

(43) Date of publication of application:
**29.01.92 Bulletin 92/05**

(45) Publication of the grant of the patent:
**22.02.95 Bulletin 95/08**

(84) Designated Contracting States:
**DE FR GB**

(56) References cited:
**EP-A- 0 350 330**

**MOLECULAR CRYSTALS AND LIOUID CRYSTALS INCORPORATING NONLINEAR OPTICS, vol. 174, Sepember 1989, pages 89-99, Gordon and Breach Science Publishers S.A., New York, US; G. GALLI et al.: "The mesomorphic behaviour of bisalkyl 4,4'-(terephthaloyldioxy)dibenzoates as models of thermotropic polyesters"**

**PATENT ABSTRACTS OF JAPAN, vol. 13, no. 151 (C-584)[3499], 12th April 1989, page 26 C 584; & JP-A-63 307 837**

(73) Proprietor: **Showa Shell Sekiyu Kabushiki Kaisha**
**2-5, Kasumigaseki 3-chome**
**Chiyoda-ku**
**Tokyo (JP)**

(72) Inventor: **Isozaki, Tadaaki, c/o SHOWA SHELL SEKIYU K.K.**
**2-5, Kasumigaseki 3-chome**
**Chiyoda-ku,**
**Tokyo 100 (JP)**
Inventor: **Mogamiya, Hiroyuki, c/o SHOWA SHELL SEKIYU K.K.**
**2-5, Kasumigaseki 3-chome**
**Chiyoda-ku,**
**Tokyo 100 (JP)**
Inventor: **Sakuma, Shigenori, c/o SHOWA SHELL SEKIYU K.K.**
**2-5, Kasumigaseki 3-chome**
**Chiyoda-ku,**
**Tokyo 100 (JP)**
Inventor: **Yamakawa, Noriko, c/o SHOWA SHELL SEKIYU K.K.**
**2-5, Kasumigaseki 3-chome**
**Chiyoda-ku,**
**Tokyo 100 (JP)**

**EP 0 468 882 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

(74) Representative: **Bourgognon, Jean-Marie et al**
**Cabinet Flechner**
**22, Avenue de Friedland**
**F-75008 Paris (FR)**

## Description

The present invention relates to liquid crystal compounds suitable to be used for electrooptical devices utilizing response of antiferroelectric liquid crystals to electric fields and in particular, to liquid crystal compounds which contain a phenylcarbonyloxy group and able to show optically tristable states.

Since liquid crystal display devices have excellent characteristics such as (1) low voltage operability, (2) low power consumption, (3) low thickness display, and (4) passive display device, TN type, STN type and Gest-Host type have been developed and put to practical use.

However, the display devices which are widely utilized in the market and use nematic liquid crystals are restricted because their response speed is as low as a few msec to some msec.

In order to solve these problems, STN type and active matrix type using a thin film transistor have been developed. Although the STN type display devices have been excellent in display qualities such as display contrast and viewing angle, there are problems that the control of cell gap or tilt angle requires a high accuracy and response is somewhat slow.

Therefore, development of novel liquid crystal display systems excellent in responsiveness has been demanded and it has been tried to develop ferroelectric liquid crystals which may make it possible to provide ultra-high speed devices having very short optical response time in the order of a few $\mu$sec.

As ferroelectric liquid crystals, DOBAMBC (p-decyloxybenzylidene-p-amino-2-methylbutyl cinnamate) is synthesized for the first time by Meyer et al. in 1975.

Further, ferroelectric liquid crystals such as DOBAMBC have attracted much attention because of their high speed response of submicro-second and memory characteristics in display devices as reported by Clark and Lagarwall in 1980. [N.A. Clark, et al., Appl. Phys. Lett., 36,899 (1980)].

However, their systems still have many technical tasks to be solved for practical use. Especially, there have been no compounds which are ferroelectric liquid crystals at room temperature and besides, there have not yet been established the methods which are effective and practical for control of liquid crystal molecular alignment which is essential for display.

Since the above report was made, many attempts have been made from both aspects of liquid crystal compounds and display devices utilizing the switching between twisted bistable states, but high contrast and proper threshold properties have not yet been obtained.

From these viewpoints, other switching systems have also been investigated and transitional scattering systems have been proposed. Thereafter, in 1988, a tristable state switching system of liquid crystals having tristable state reported [A.D.L. Chandani, T. Hagiwara, Y.Suzuki et al., Japan. J. of Appl. Phys., 27, (5), L729-L732 (1988)].

The term "tristable states" means that,in an electrooptical device where antiferroelectric liquid crystals are laid between the first electrode substrate plate and the second electrode substrate plate which is apart at a given space from the first one and it is constructed so that an electric voltage for electric field is applied to both the first and second electrode substrate plates, when voltage in the form of a triangular wave as shown in Fig. 1 A is applied, the antiferroelectric liquid crystals have molecular orientation of the first stable state (as shown in Fig. 1 D-2) where no electric field is applied to, but upon application of electric field, molecular orientation of the second stable state (as shown in Fig. 1 D-1) which is different from the first stable state in one of electric field directions and molecular orientation of the third stable state (as shown in Fig. 1 D-3) which is different from the first and second stable states in the other direction of electric field, as shown in Fig. 1 D.

The tristable state switching system employs clear threshold specificity and hysteresis to driving voltage exhibited by liquid crystal phase S*(3) having tristable states fundamentally different from the conventional bistable states in molecular orientation of liquid crystals. This is considered to be an epoch-making method by which a moving image of large scale can be displayed by simple matrix system. (Japanese Patent Kokai No. Hei 2-153322).

Liquid crystal compounds which are able to exhibit tristable states when they are in phase S*(3) are disclosed in Japanese Patent Kokai Nos. Hei 1-316339, 1-316367, 1-316372, 2-28128 and 1-213390, and in European Patent application EP-A-350330.

As a result of intensive research conducted by the inventors on ester type liquid crystal compounds in view of the above-mentioned problems, it has been found that liquid crystal compounds containing a phenylcarbonyloxy group are chemically and photochemically stable and have large dielectric anisotropy and besides, have the liquid crystal phase S*(3) having tristable states, which has not been obtained in the conventional ferroelectric liquid crystals.

That is, an object of the present invention is to provide novel liquid crystal compounds having phenyl ester groups, which are expected to be applicable to new electrooptical devices and liquid crystal display

which utilize the liquid crystal phase S*(3) having tristable states.

Fig. 1 A shows triangular wave applied, and Figs. 1 B, 1 C and 1 D show optical responses of commercially available nematic liquid crystals, bistable states liquid crystals, and the present tristable state liquid crystals, respectively.

Fig. 2 shows infrared absorption spectrum of the compound of the present invention obtained in Example 2.

Fig. 3 shows infrared absorption spectrum of the compound of the present invention obtained in Example 3.

The first of the present invention relates to an optically active liquid crystal compound represented by the formula [I]:

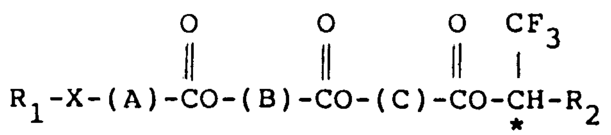 .....[I]

wherein $R_1$ and $R_2$ each represents an alkyl group of 4 - 18 carbon atoms, X represents O, COO or a single bond, and (A), (B) and (C) each represents

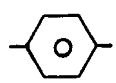 , 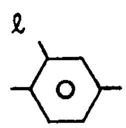 or 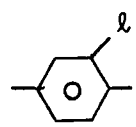

in which ℓ represents a halogen atom.

The second of the present invention relates to an optically active liquid crystal compound represented by the formula [II]:

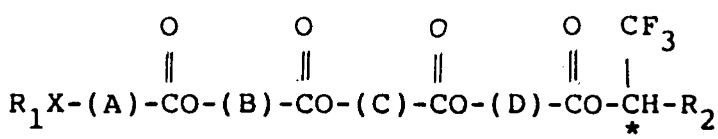 ....[II]

wherein $R_1$, $R_2$ and X each has the same meaning as above and (A), (B), (C) and (D) each represents

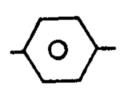 , 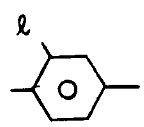 or 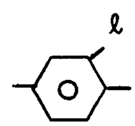

in which ℓ represents a halogen atom.

The third of the present invention relates to an optically active liquid crystal compound represented by the formula [III]:

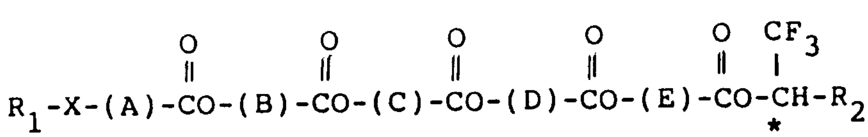.... [III]

wherein $R_1$, $R_2$ and X each has the same meaning as above and (A), (B), (C), (D) and (E) each represents

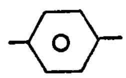 , 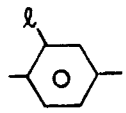 or 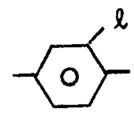

in which ℓ represents a halogen atom.

The fourth of the present invention relates to an optically active liquid crystal compound having optically tristable states represented by the formula [IV]:

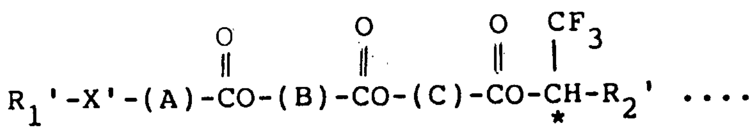 .... [IV]

wherein $R_1'$ and $R_2'$ each represents a straight chain alkyl group of 5 - 16 carbon atoms, X′ represents O or COO and (A), (B), (C) and ℓ each has the same meaning as above.

The fifth of the present invention relates to an optically active liquid crystal compound having optically tristable states represented by the formula [V]:

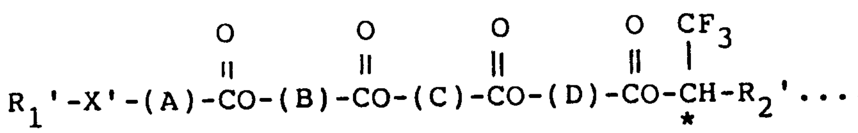 .... [V]

wherein $R_1'$, $R_2'$, X′, (A), (B), (C), (D) and ℓ each has the same meaning as above.

The sixth of the present invention relates to an optically active liquid crystal compound having optically tristable states represented by the formula [VI]:

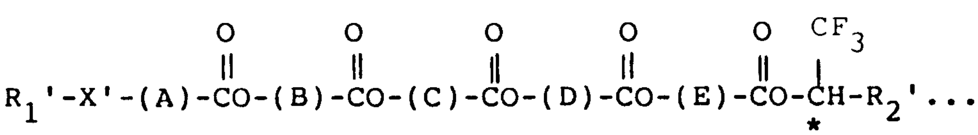 ... [VI]

wherein $R_1'$, $R_2'$, X', (A), (B), (C), (D), (E) and each has the same meaning as above and $R_1'$ and $R_2'$ each is preferably a straight chain alkyl group of 6 - 12 carbon atoms and ℓ is preferably F or Cl.

One example of processes for preparing the compounds is shown below.

(1) A fatty acid chloride is allowed to react with p-hydroxybenzoic acid in the presence of triethylamine to obtain a 4-alkylcarbonyloxybenzoic acid, which is then converted to a 4-alkylcarbonyloxybenzoic acid chloride with excess thionyl chloride. This chloride is allowed to react with p-hydroxybenzoic acid in the presence of triethylamine to obtain a 4-[(4-alkylcarbonyloxyphenyl)carbonyloxy]benzoic acid, which is then converted to a 4-(4-alkylcarbonyloxyphenylcarbonyloxy)benzoic acid chloride with excess thionyl chloride.

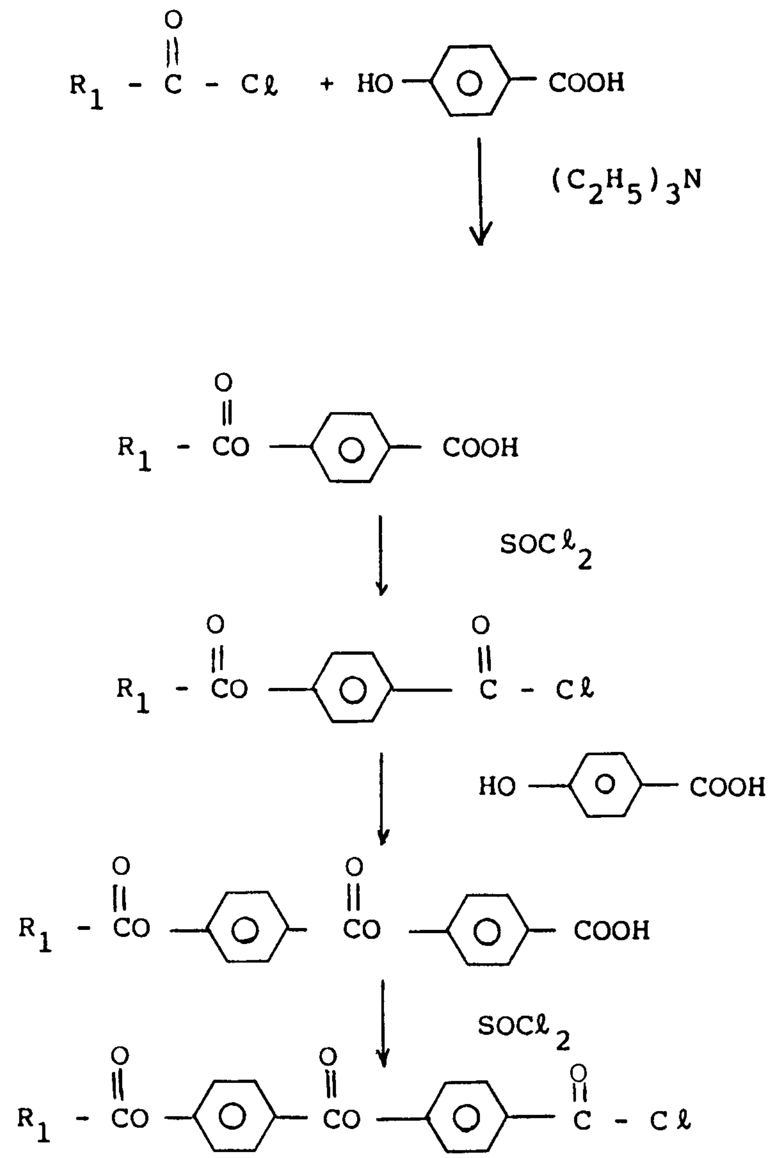


(2) 4-Benzyloxybenzoic acid chloride is allowed to react with an optically active 1,1,1-trifluoro-2-alkanol in the presence of triethylamine to obtain a 1,1,1-trifluoro-2-alkyl 4-benzyloxybenzoate, which is subjected to hydrogenolysis reaction to obtain a 1,1,1-trifluoro-2-alkyl 4-hydroxybenzoate.

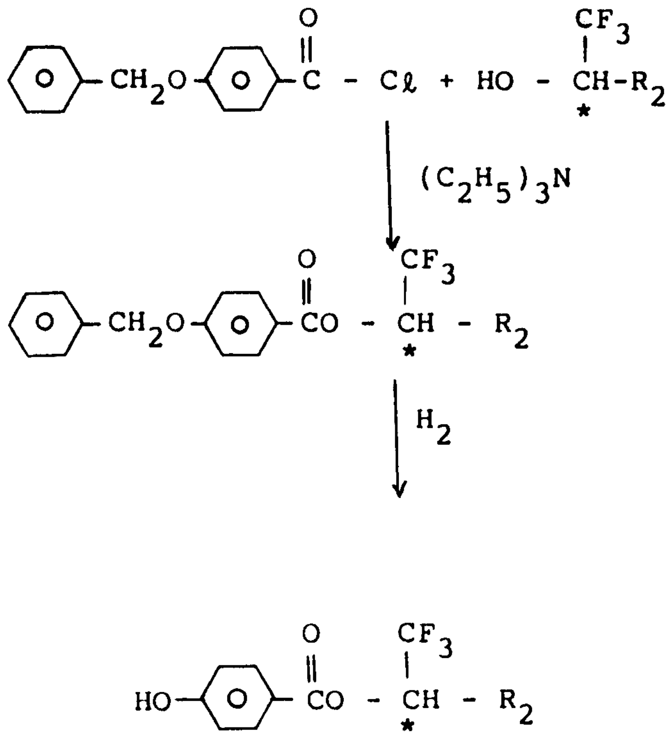


(3) The chloride obtained in (1) is allowed to react with the phenol obtained in (2) in the presence of triethylamine to obtain a 4-(1,1,1-trifluoro-2-alkyloxycarbonyl)phenyl 4-[(4-alkylcarbonyloxy)-phenylcarbonyloxy]benzoate.

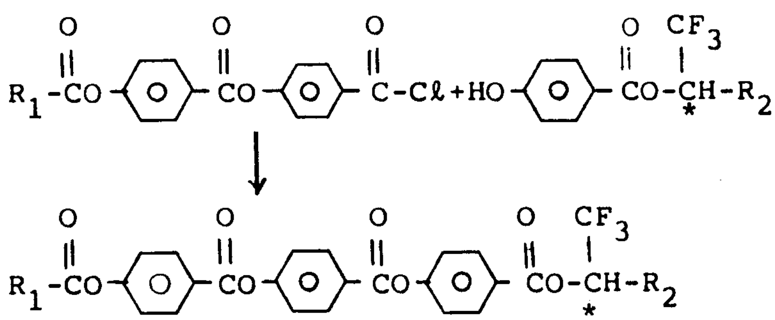

p-Alkyloxybenzoic acid is allowed to react with thionyl chloride to produce p-alkyloxybenzoic acid chloride, which is then allowed to react with p-hydroxybenzoic acid in the presence of triethyl amine to produce 4-(4-alkyloxyphenylcarbonyloxy)benzoic acid. The product is then allowed to react with thionyl chloride to produce 4-(4-alkyloxyphenylcarbonyloxy)benzoic acid chloride, which is allowed to react with 1,1,1-trifluoro-2-alkyl 4-hydroxybenzoate obtained (2) above to produce 4-(1,1,1-trifluoro-2-alkyloxy-carbonyl)phenyl 4-[(4-alkyloxy)phenylcarbonyloxy] benzoate.

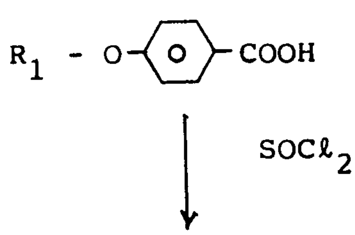

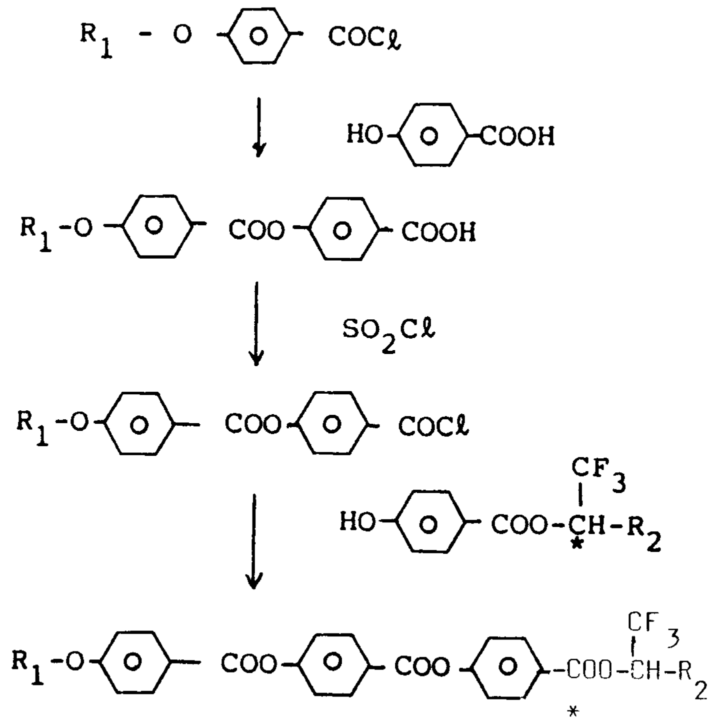


Example 1

(1) Synthesis of 1,1,1-trifluoro-2-octyl 4-benzyloxybenzoate:

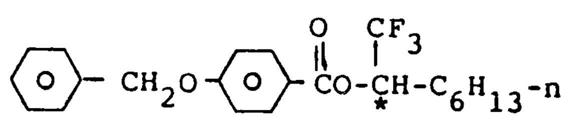

To a solution of 4-benzyloxybenzoic acid chloride (4.3 g) in methylene chloride (50 ml) was gradually added under ice cooling a solution of optically active 1,1,1-trifluoro-2-octanol (2.9 g), dimethylaminopyridine (0.6 g) and triethylamine (1.7 g) in methylene chloride (50 ml).

After the reaction mixture was left to stand to reach room temperature, reaction was allowed to proceed for 24 hours. The reaction mixture was poured in ice water and was extracted with methylene chloride. The methylene chloride layer was washed with dilute hydrochloric acid, water, 1N aqueous sodium carbonate solution, and water in this order and was dried over anhydrous magnesium sulfate. The solvent was distilled off to obtain a crude product. The product was subjected to toluene-silica gel column chromatography and was further recrystallized from ethanol to obtain the titled compound (3.8 g).

(2) Synthesis of 1,1,1-trifluoro-2-octyl 4-hydroxybenzoate:

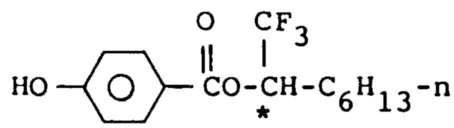

To a solution of the compound obtained in (1) above in methanol (100 ml) was added 10% Pd carried on carbon (0.4 g) and the mixture was subjected to hydrogenolysis reaction in a hydrogen atmosphere to obtain the titled compound (2.8 g).

(3) Synthesis of 4-n-decanoyloxybenzoic acid:

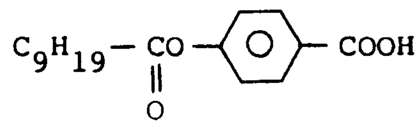

To a solution of p-hydroxybenzoic acid (3 g) and triethylamine (2.4 g) in dichloromethane (30 ml) were added decanoyl chloride (4.3 g) and dimethylaminopyridine (0.2 g) and the mixture was stirred for about 20 hours at room temperature. After dilute hydrochloric acid was added to the mixture, the organic layer was separated by a separating funnel. The solvent was distilled off and the residue was washed with n-hexane and then dried to obtain the titled compound (about 5 g).

(4) Synthesis of 4-n-decanoyloxybenzoic acid chloride:

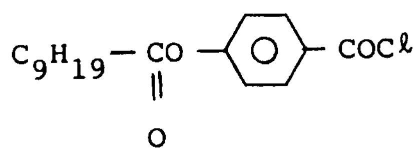

4-n-Decanoyloxybenzoic acid (5.0 g) obtained in (3) above was added to thionyl chloride (10 g) and a slight amount of N,N-dimethylformamide was added thereto, followed by refluxing for 4 hours. Excess thionyl chloride was distilled off to obtain the titled compound (5.2 g).

(5) Synthesis of 4-carboxyphenyl 4-n-decanoyloxy-benzoate:

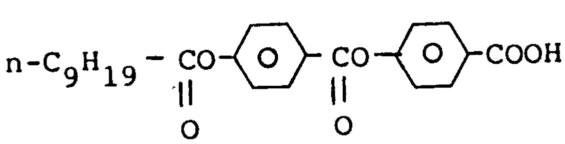

To a solution of p-hydroxybenzoic acid (2 g) and triethylamine (1.3 g) in methylene chloride (30 ml) were added 4-n-decanoyloxybenzoic acid chloride (4 g) obtained in (4) above and dimethylaminopyridine (0.3 g). The mixture was stirred at room temperature for about 20 hours. After dilute hydrochloric acid was added thereto, the organic layer was separated by a separating funnel. The solvent was distilled off and the residue was washed with n-hexane and then dried to obtain the titled compound (about 3 g).

(6) Synthesis of 4-[(4-n-decanoyloxyphenyl)-carbonyloxy]benzoic acid chloride:

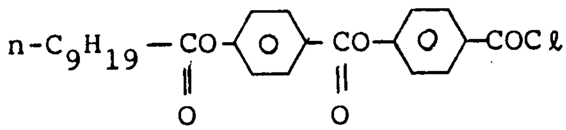

4-Carboxyphenyl 4-n-decanoyloxybenzoate obtained in (5) above (3 g) was added to thionyl chloride (about 10 g) and a very small amount of N,N-dimethylformamide was added thereto, followed by refluxing for 4 hours. Excess thionyl chloride was distilled off to obtain the titled compound (about 3.1 g).

(7) Synthesis of 4-(1,1,1-trifluoro-2-octyloxycarbonyl)phenyl 4-[(4-n-decanoyloxy)-phenylcarbonyloxy]-benzoate

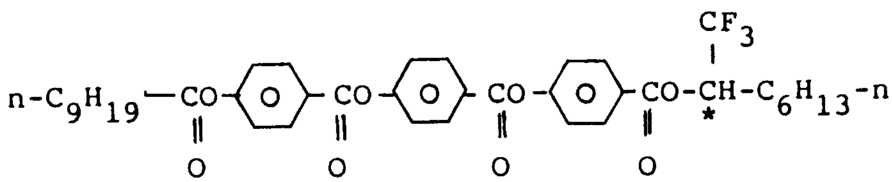

To a solution of 1,1,1-trifluoro-2-octyl 4-hydroxybenzoate obtained in (2) above (0.5 g) and triethylamine (0.16 g) in methylenechloride (30 ml) was gradually added dropwise a solution of 4-[(4-n-decanoyloxyphenyl)carbonyloxy] benzoic acid chloride obtained in (6) above (0.7 g) in methylene chloride (30 ml). Furthermore, dimethylaminopyridine (0.05 g) was added thereto and the mixture was stirred at room temperature for 24 hours. The reaction mixture was poured in water and the solution was made neutral. Then, the methylene chloride layer was separated and dried over anhydrous magnesium sulfate. Then, methylene chloride was distilled off. The residue was purified by silica gel column chromatography (developer: hexane/ethyl acetate = 20/1) to obtain the titled compound (0.11 g).

Specific rotation $[\alpha]_D^{20}$ = +25.5°

Phase transition temperatures (°C) which were observed under a polarizing microscope using a hot stage were

$$\text{Cry} \xleftarrow{19.4} \text{S*(3)} \xleftarrow{99.0} \text{SA} \xleftarrow{120.1} \text{Iso}$$

where

S*(3): tristable state liquid crystal phase

Example 2

(1) Synthesis of 1,1,1-trifluoro-2-octyl-2-fluoro-4-benzyloxybenzoate:

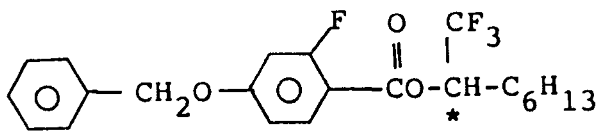

To a solution of 2-fluoro-4-benzyloxy-benzoic acid chloride (2.7 g) in methylene chloride (30 ml) was gradually added under ice cooling a solution of optically active 1,1,1-trifluoro-2-octanol (1.8 g), dimethylaminopyridine (0.3 g) and triethylamine (1.5 g) in methylene chloride (50 ml).

After the reaction mixture was left to stand to reach room temperature, a reaction was allowed to proceed for 24 hours. The reaction mixture was poured in ice water and was extracted with methylene chloride. The extracted methylene chloride layer was washed with dilute hydrochloric acid, water, 1N aqueous sodium carbonate solution, and water in this order and was dried over anhydrous magnesium sulfate. The solvent was distilled off to obtain a crude product. The product was subjected to toluene-silica gel column chromatography and was further recrystallized from ethanol to obtain the titled compound (2.5 g).

(2) Synthesis of 1,1,1-trifluoro-2-octyl 2-fluoro-4-hydroxybenzoate:

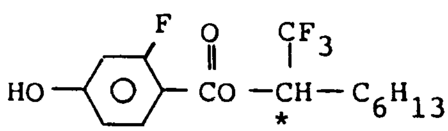

To a solution of the compound obtained in (1) above in methanol (100 ml) was added 10% Pd carried on carbon (0.25 g) and the mixture was subjected to hydrogenolysis reaction in a hydrogen atmosphere to obtain the titled compound (1.8 g).

(3) Synthesis of 3-fluoro-4-(1,1,1-trifluoro-2-octyloxycarbonyl)phenyl 4-[(4-n-decanoyloxy)-phenylcarbonyloxy]benzoate

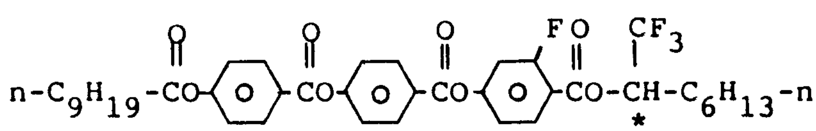

To a solution of 1,1,1-trifluoro-2-octyl 2-fluoro-4-hydroxybenzoate obtained in (2) above (0.5 g) and triethylamine (0.16 g) in methylene-chloride (30 ml) was gradually added dropwise a solution of 4-[(4-n-decanoyloxyphenyl)carbonyloxy] benzoic acid chloride obtained in (6) of Example 1 (0.7 g) in methylene chloride (30 ml). Furthermore, dimethylaminopyridine (0.05 g) was added thereto and the mixture was stirred at room temperature for 24 hours. The reaction mixture was poured in water and the solution was made neutral. Then, the methylene chloride layer was separated and dried over anhydrous magnesium sulfate. Then, methylene chloride was distilled off. The residue was purified by silica gel column chromatography (developer: hexane/ethyl acetate = 20/1) to obtain the titled compound (0.13 g).

Specific rotation $[\alpha]_D^{20}$ = +23.8°

Phase transition temperatures (°C) which were observed under a polarizing microscope using a hot

stage were

Cry ← S*(3) ← S*c ← SA ← Iso
15 101.5 107 118.5

where

S*(3): tristable state liquid crystal phase

IR spectrum of the titled compound is shown in Fig. 2.

Example 3

Synthesis of 4-(1,1,1-trifluoro-2-octyloxycarbonyl)phenyl 2-fluoro-4-[(4-n-decanoyloxy)phenylcarbonyloxy]-benzoate :

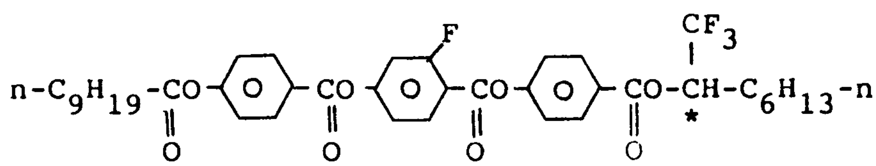

Example 1 was repeated except that 2-fluoro-4-hydroxybenzoic acid was used in place of the p-hydroxybenzoic acid used in (5) of Example 1 to obtain the titled compound (0.08 g).

Phase transition temperatures (°C) which were observed under a polarization microscope using a hot stage were

Cry ← S*(3) ← S*(4) ← S*c ← SA ← Iso
18 91 94 95 116.4

wherein

S*(3): tristable state liquid crystal phase

S*(4): tetrastable state liquid crystal phase

IR spectrum of the titled compound is shown in Fig. 3.

Example 4

Synthesis of 3-fluoro-4-(1,1,1-trifluoro-2-octyloxycarbonyl)phenyl 2-fluoro-4-[(4-n-decanoyloxy)-phenylcarbonyloxy]benzoate:

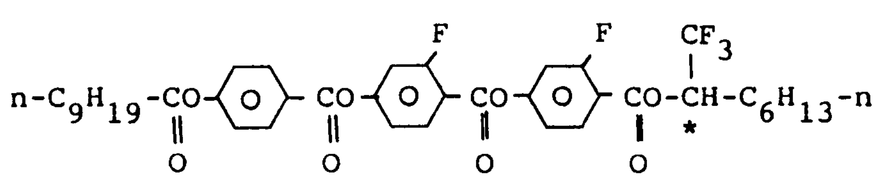

Example 1 was repeated except that 2-fluoro-4-hydroxybenzoic acid was used in place of the p-hydroxybenzoic acid used in (5) of Example 1 and 2-fluoro-4-benzyloxybenzoic acid chloride was used in place of the 4-benzyloxybenzoic acid chloride used in (1) of Example 1 to obtain the titled compound (0.02

g).

Phase transition temperatures (°C) which were observed under a polarization microscope using a hot stage were

Cry ← Sx ← S*(3) ← S*c ← SA ← Iso

-25 -5 97 103 115.8

where

S*(3): tristable state liquid crystal phase

Sx: A liquid crystal phase having field response, which has higher numbers of stable state.

The novel liquid crystal compounds of the present invention all have tristable states and have a wide variety of applications such as display devices and switching devices.

Example 5

(1) Synthesis of 4-n-octyloxybenzoic acid chloride:

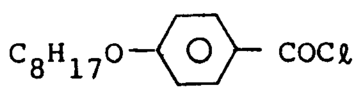

4-n-Octyloxybenzoic acid (4.3 g) was added to thionyl chloride (10 g) and a slight amount of N,N-dimethylformamide was added thereto, followed by refluxing for 4 hours. Unaltered thionyl chloride was distilled off to obtain the titled compound (4.5 g).

(2) Synthesis of 4-carboxyphenyl 4-n-octylbenzoate:

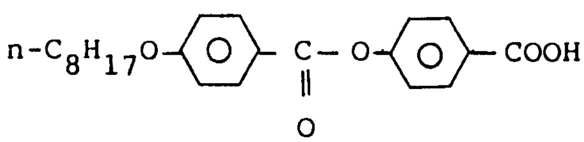

To a solution of p-hydroxybenzoic acid (2 g) and triethylamine (1.3 g) in methylene chloride (30 ml) were added 4-n-octyloxybenzoic acid chloride (3.4 g) obtained in (4) of example 1 and dimethylaminopyridine (0.3 g). The mixture was stirred at room temperature for about 20 hours. After dilute hydrochloric acid was added thereto, the organic layer was separated by a separating funnel. The solvent was distilled off and the residue was washed with n-hexane and then dried to obtain the titled compound (about 2.7 g).

(3) Synthesis of 4-[(4-n-octyloxyphenyl)carbonyloxy]benzoic acid chloride:

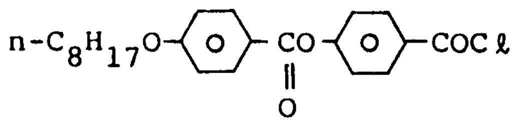

4-Carboxyphenyl 4-n-octyloxybenzoate obtained in (2) above (2.7 g) was added to thionyl chloride (about 10 g) and a very small amount of N,N-dimethylformamide was added thereto, followed by refluxing for 4 hours. Unaltered thionyl chloride was distilled off to obtain the titled compound (about 3.1 g).

(4) Synthesis of 4-(1,1,1-trifluoro-2-octyloxycarbonyl)phenyl 4 [(4-n-octyloxy)phenyl-carbonyloxy]benzoate

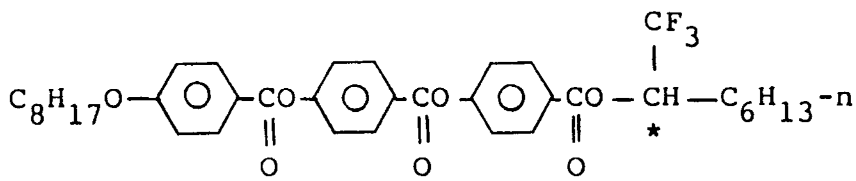

To a solution of 1,1,1-trifluoro-2-octyl 4-hydroxybenzoate obtained in (2) of example 1 above (0.5 g) and triethylamine (0.16 g) in methylenechloride (30 ml) was gradually added dropwise a solution of 4-[(4-n-octyloxyphenyl)carbonyloxy]benzoic acid chloride obtained in (3) above (0.6 g) in methylene chloride (30 ml). Furthermore, dimethylaminopyridine (0.05 g) was added thereto and the mixture was stirred at room temperature for 24 hours. The reaction mixture was poured in water and the solution was made neutral. Then, the methylene chloride layer was separated and dried over anhydrous magnesium sulfate. Then, methylene chloride was distilled off. The residue was purified by silica gel column chromatography (developer: hexane/ethyl acetate = 20/1) to obtain the titled compound (0.10 g).

Specific rotation $[\alpha]_D^{20}$ = +25.5°

Phase transition temperatures (°C) which were observed under a polarization microscope using a hot stage were

$$\text{Cry} \underset{61.2}{\overset{83.6}{\rightleftarrows}} \text{S*(3)} \underset{99.0}{\overset{100.3}{\rightleftarrows}} \text{SA} \underset{123.2}{\overset{121.9}{\rightleftarrows}} \text{Iso}$$

where

S*(3): tristable state liquid crystal phase.

## Claims

**1.** An optically active liquid crystal compound represented by the formula [I]:

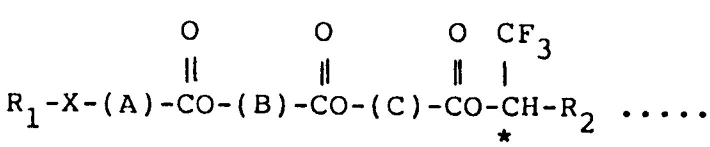 ..... [I]

wherein $R_1$ and $R_2$ each represents an alkyl group of 4 - 18 carbon atoms, X represents O, COO or a single bond, and (A), (B) and (C) each represents

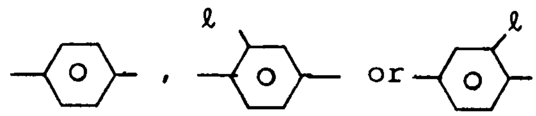

, or

in which ℓ represents a halogen atom.

**2.** An optically active liquid crystal compound represented by the formula [II]:

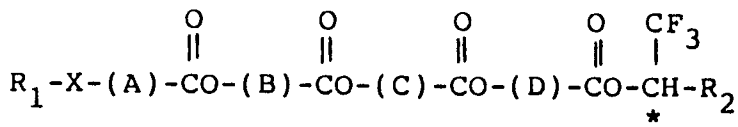
....[II]

wherein $R_1$, $R_2$ and X each has the same meaning as above and (A), (B), (C) and (D) each represents

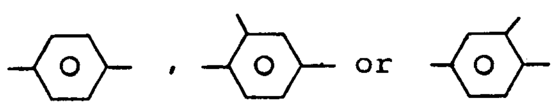

, or

in which ℓ represents a halogen atom.

**3.** An optically active liquid crystal compound represented by the formula [III]:

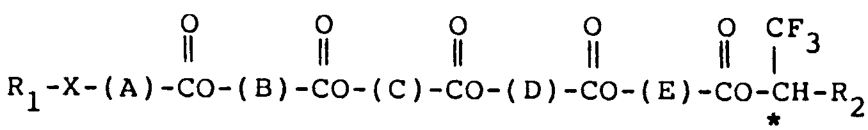
....[III]

wherein $R_1$, $R_2$ and X each has the same meaning as above and (A), (B), (C), (D) and (E) each represents

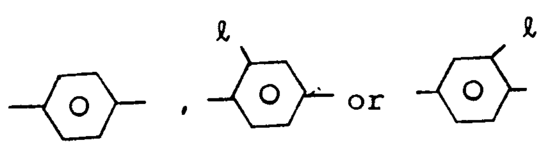

, or

in which ℓ represents a halogen atom.

**4.** An optically active liquid crystal compound which is able to exhibit tristable states represented by the formula [IV]:

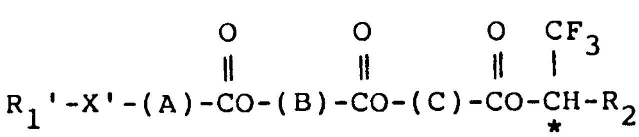
.....[IV]

wherein $R_1'$ and $R_2'$ each represents a straight chain alkyl group of 5 - 16 carbon atoms, X' represents O or COO and (A), (B), (C) and ℓ each has the same meaning as above.

**5.** An optically active liquid crystal compound which is able to exhibit tristable states, represented by the formula [V]:

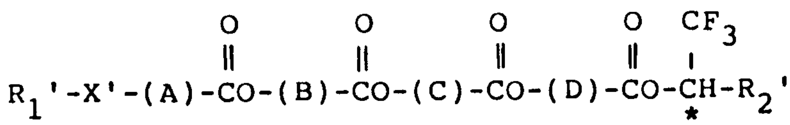

... [V]

wherein $R_1'$, $R_2'$, X', (A), (B), (C), (D) and ℓ each has the same meaning as above.

**6.** An optically active liquid crystal compound which is able to exhibit tristable states, represented by the formula [VI]:

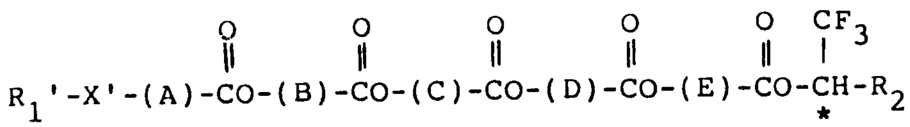

... [VI]

wherein $R_1'$, $R_2'$, X', (A), (B), (C), (D), (E) and ℓ each has the same meaning as above.

**7.** An optically active liquid crystal compound of claim 1, which has tristable states when it is in S*(3) phase.

## Patentansprüche

**1.** Durch Formel [I] wiedergegebene, optisch aktive Flüssigkristall-Verbindung:

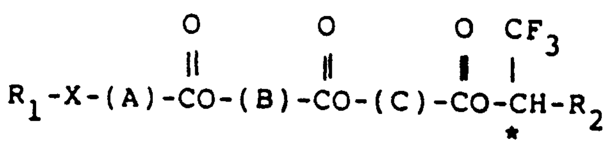

[I]

worin $R_1$ und $R_2$ jeweils für eine Alkylgruppe mit 4 bis 18 Kohlenstoffatomen stehen, X für O, COO oder eine Einfachbindung steht und (A), (B) und (C) jeweils stehen für

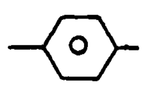 , 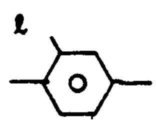 oder 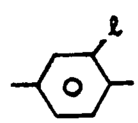

worin ℓ für ein Halogenatom steht.

2. Durch Formel [II] wiedergegebene optisch aktive Flüssigkristall-Verbindung:

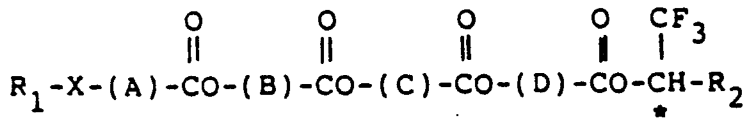

[II]

worin $R_1$, $R_2$ und X jeweils die gleiche Bedeutung wie oben haben und (A), (B), (C) und (D) jeweils stehen für

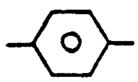 , 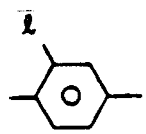 oder 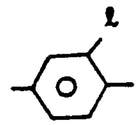

worin ℓ für ein Halogenatom steht.

3. Durch Formel [III] wiedergegebene optisch aktive Flüssigkristall-Verbindung:

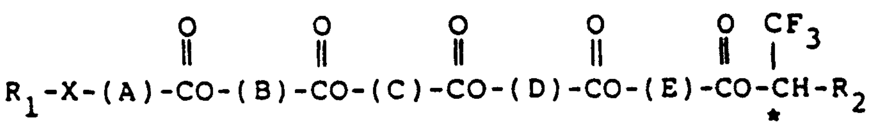

[III]

worin $R_1$, $R_2$ und X jeweils dieselbe Bedeutung wie oben aufweisen und (A), (B), (C), (D) und (E) jeweils stehen für

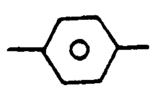 , 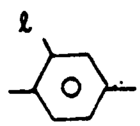 oder 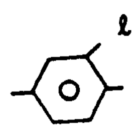

worin ℓ für ein Halogenatom steht.

4. Optisch aktive Flüssigkristall-Verbindung, die in der Lage ist, tristabile Zustände zu zeigen und wiedergegeben wird durch Formel [IV]:

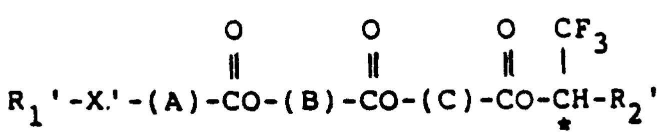

[IV]

worin $R_1$' und $R_2$' jeweils stehen für eine geradkettige Alkylgruppe mit 5 bis 16 Kohlenstoffatomen, X' für O oder COO steht und (A), (B), (C) und ℓ jeweils dieselbe Bedeutung wie oben haben.

5. Optisch aktive Flüssigkristall-Verbindung, die in der Lage ist, tristabile Zustände zu zeigen und wiedergegeben wird durch Formel [V]:

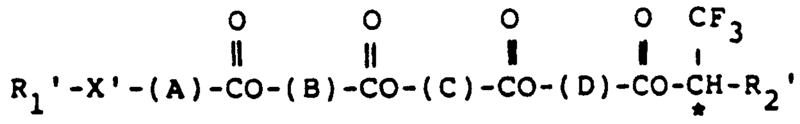

[V]

worin R₁', R₂', X', (A), (B), (C), (D) und ℓ jeweils dieselben Beutungen haben wie oben.

6. Optisch aktive Flüssigkristall-Verbindung, die in der Lage ist, tristabile Zustände zu zeigen, und wiedergegeben wird durch Formel [VI]:

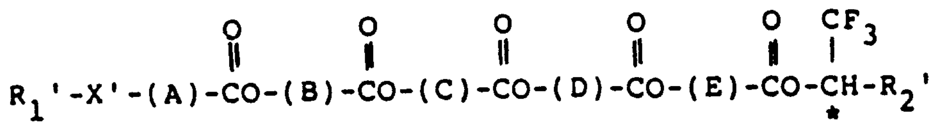

[VI]

worin R₁', R₂', X', (A), (B), (C), (D), (E) und ℓ jeweils dieselben Bedeutungen haben wie oben.

7. Optisch aktive Flüssigkristall-Verbindung nach Anspruch 1, die tristabile Zustände aufweist, wenn sie in der S*(3)-Phase vorliegt.

**Revendications**

1. Cristal liquide optiquement actif, représenté par la formule (I) :

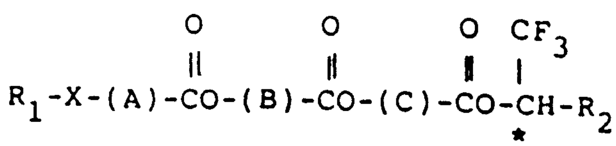

..... (I)

dans laquelle $R_1$ et $R_2$ représentent chacun un groupe alcoyle de 4 à 18 atomes de carbone, X représente O, COO ou une liaison simple, et (A), (B) et (C) représentent chacun

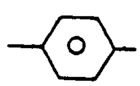 , 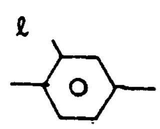 ou 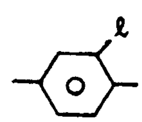

dans lesquels ℓ représente un atome d'halogène.

2. Cristal liquide optiquement actif, représenté par la formule (II) :

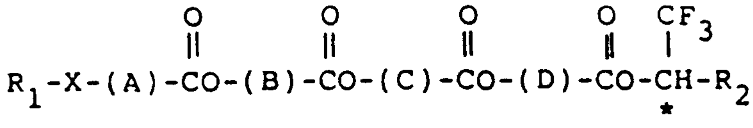

.... (II)

dans laquelle $R_1$, $R_2$ et X ont chacun la même signification que ci-dessus et (A), (B), (C) et (D) représentent chacun

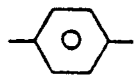 , 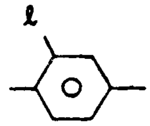 ou 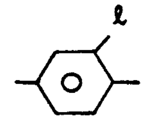

dans lesquels ℓ représente un atome d'halogène.

**3.** Cristal liquide optiquement actif, représenté par la formule (III) :

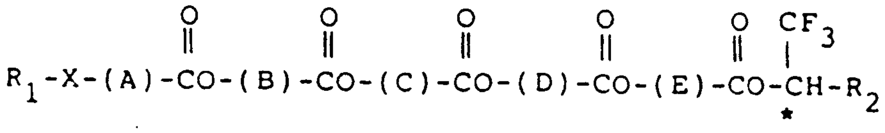 ....(III)

dans laquelle $R_1$, $R_2$ et X ont chacun la même signification que ci-dessus et (A), (B), (C), (D) et (E) représentent chacun

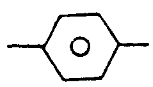 , 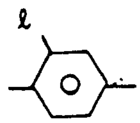 ou 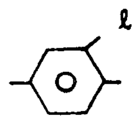

dans lesquels ℓ représente un atome d'halogène.

**4.** Cristal liquide optiquement actif qui est apte à présenter des états tristables, représenté par la formule (IV) :

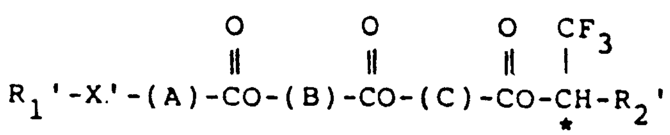 ..... (IV)

dans laquelle $R_1$' et $R_2$' représentent chacun un groupe alcoyle linéaire de 5 à 16 atomes de carbone, X' représente O ou COO et (A), (B), (C) et ℓ ont chacun la même signification que ci-dessus.

**5.** Cristal liquide optiquement actif qui est apte à présenter des états tristables, représenté par la formule (V):

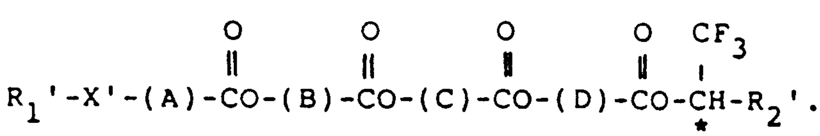 ...(V)

dans laquelle $R_1$', $R_2$', X', (A), (B), (C), (D) et ℓ ont chacun la même signification que ci-dessus.

**6.** Cristal liquide optiquement actif qui est apte à présenter des états tristables, représenté par la formule (VI) :

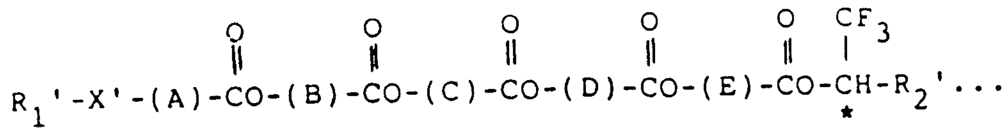 (VI)

dans laquelle $R_1'$, $R_2'$, X', (A), (B), (C), (D), (E) et ℓ ont chacun la même signification que ci-dessus.

**7.** Cristal liquide optiquement actif suivant la revendication 1, qui a des états tristables quand il est dans la phase S*(3).

FIG. 1

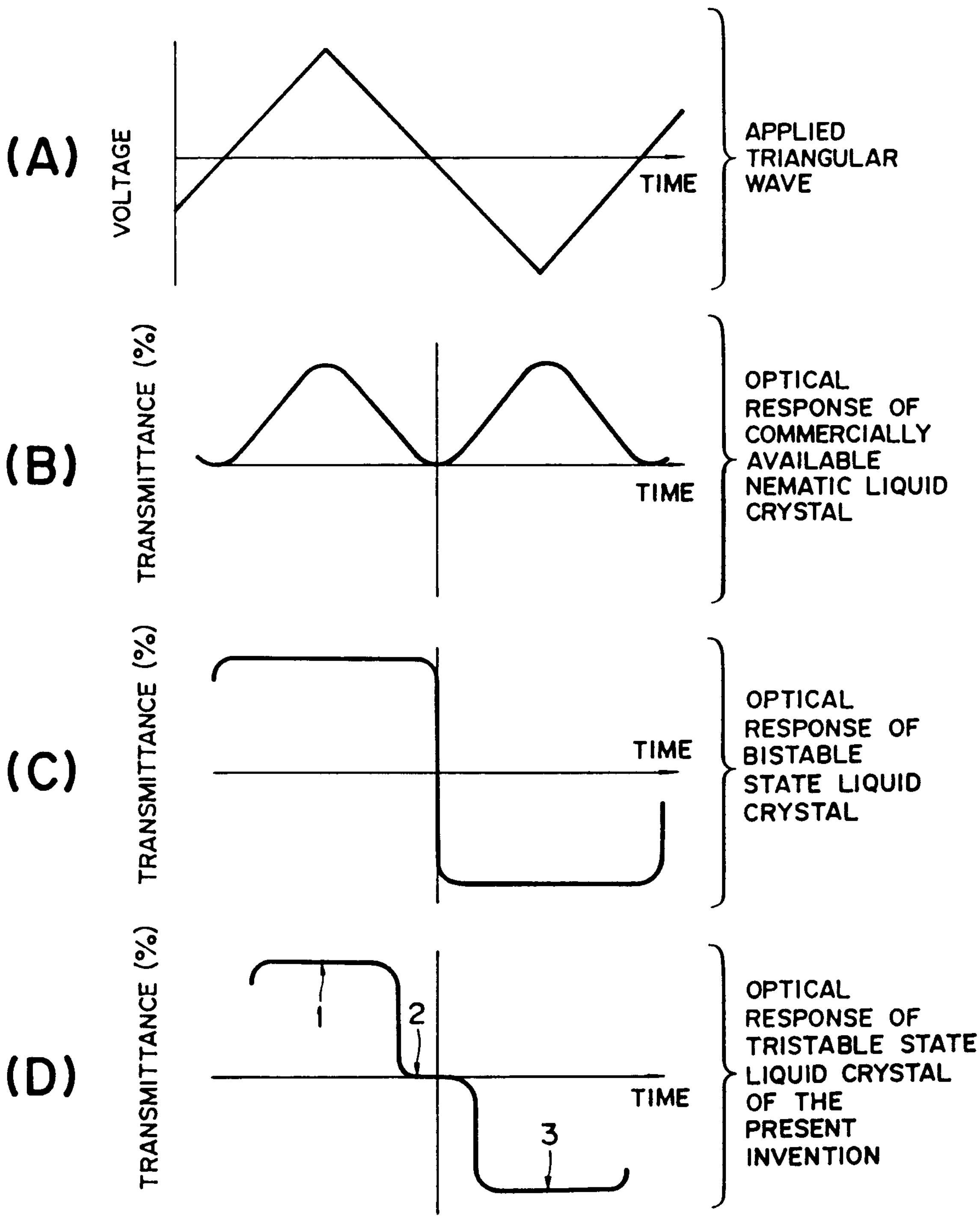
(A)
VOLTAGE
TIME
APPLIED TRIANGULAR WAVE
(B)
TRANSMITTANCE (%)
TIME
OPTICAL RESPONSE OF COMMERCIALLY AVAILABLE NEMATIC LIQUID CRYSTAL
(C)
TRANSMITTANCE (%)
TIME
OPTICAL RESPONSE OF BISTABLE STATE LIQUID CRYSTAL
(D)
TRANSMITTANCE (%)
1
2
3
TIME
OPTICAL RESPONSE OF TRISTABLE STATE LIQUID CRYSTAL OF THE PRESENT INVENTION

FIG. 2

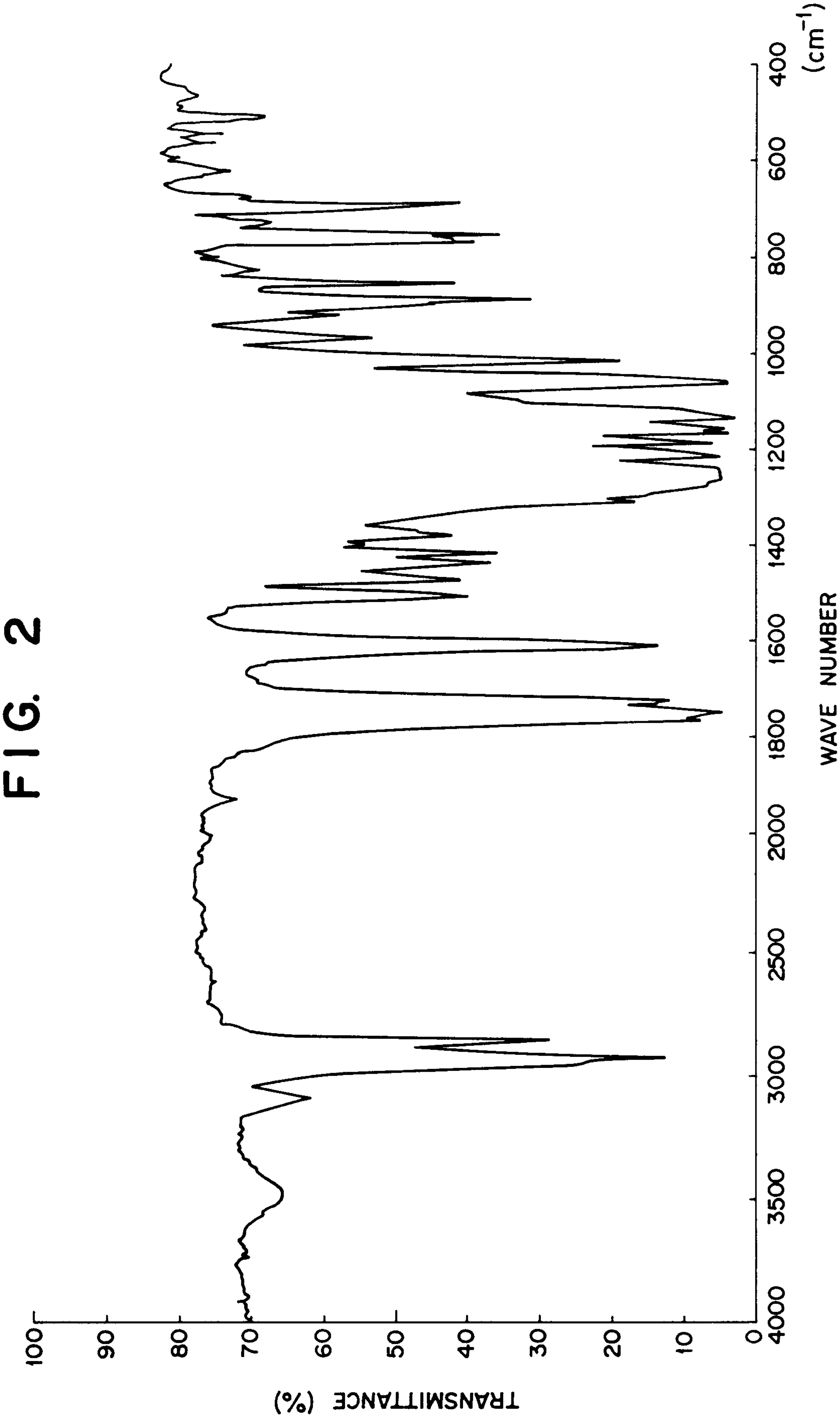
TRANSMITTANCE (%)
100
90
80
70
60
50
40
30
20
10
0
4000
3500
3000
2500
2000
1800
1600
1400
1200
1000
800
600
400
(cm-1)
WAVE NUMBER

FIG. 3

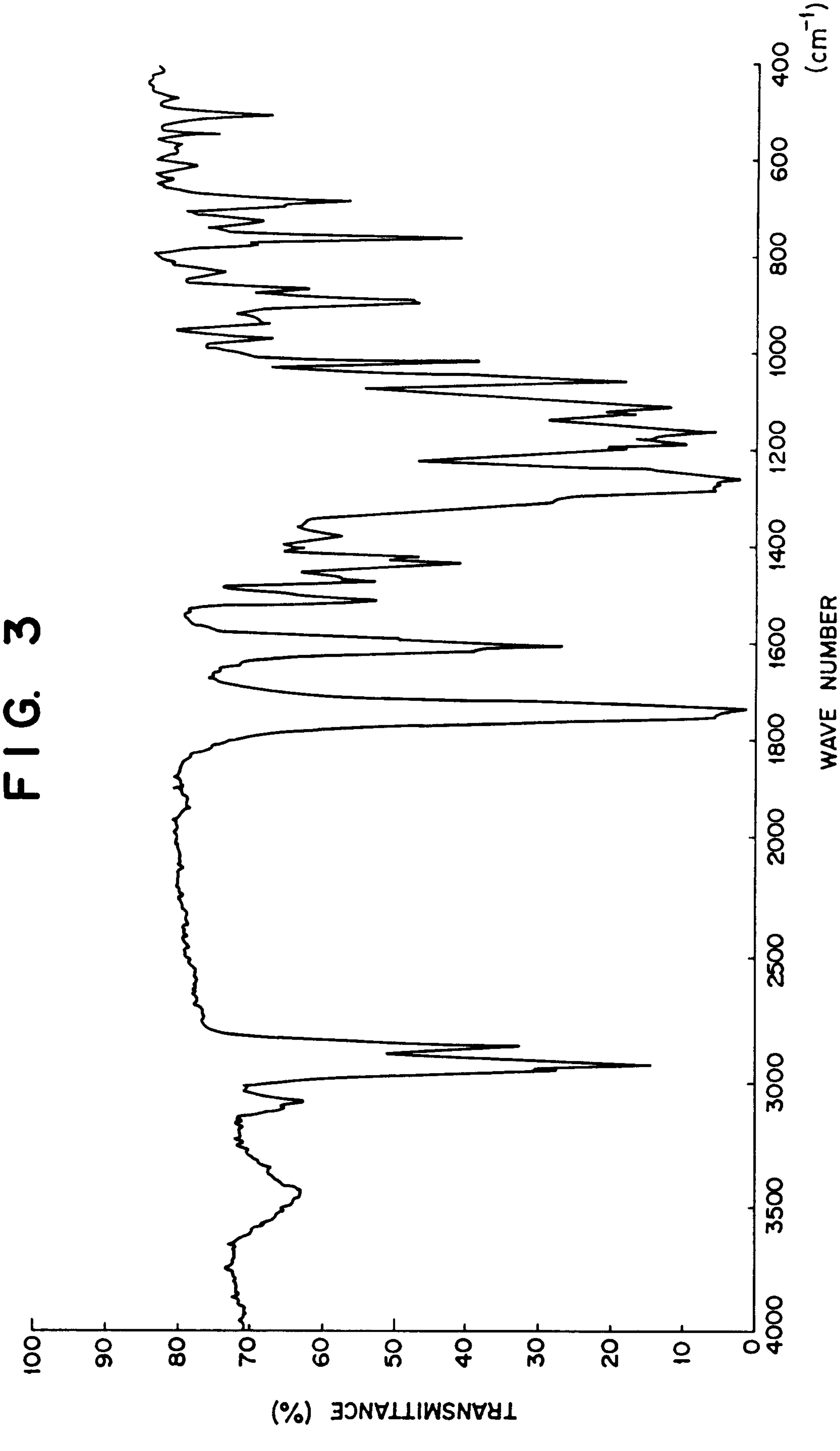
TRANSMITTANCE (%)
100
90
80
70
60
50
40
30
20
10
0
4000
3500
3000
2500
2000
1800
1600
1400
1200
1000
800
600
400
(cm⁻¹)
WAVE NUMBER